(19) Europäisches Patentamt
European Patent Office
Office européen des brevets

(11) Veröffentlichungsnummer : **0 220 592**
**B1**

# (12) EUROPÄISCHE PATENTSCHRIFT

(45) Veröffentlichungstag der Patentschrift : **01.02.89**

(51) Int. Cl.⁴ : **C 07 D319/06**

(21) Anmeldenummer : **86114181.0**

(22) Anmeldetag : **14.10.86**

## (54) 2-(3-Brompropyl)-5,5-dimethyl-1,3-dioxan, sowie Verfahren zur Herstellung desselben und des 2-(4-Brombutyl)-5,5-dimethyl-1,3-dioxans.

(30) Priorität : **19.10.85 DE 3537289**

(43) Veröffentlichungstag der Anmeldung : **06.05.87 Patentblatt 87/19**

(45) Bekanntmachung des Hinweises auf die Patenterteilung : **01.02.89 Patentblatt 89/05**

(84) Benannte Vertragsstaaten : **CH DE FR GB IT LI**

(56) Entgegenhaltungen :
**CHEMICAL ABSTRACTS, Band 83, Nr. 21, 24. November 1975, Columbus, Ohio, USA; FLEMING, I.; LONG, W.E.: "Protected 5-hydroxy pentanal synthon", Seite 568, Spalte 1, Zusammenfassung Nr. 178949t**
**CHEMICAL ABSTRACTS, Band 88, Nr. 19, 8. Mai 1978, Columbus, Ohio, USA; FORBES, C.P.; WENTELER, G.L.; WIECHERS, A.: "An improved method for the acetal substituted Grignard reagents in organic systesis", Seite 529, Spalte 2, Zusammenfassung Nr. 136535c**

(73) Patentinhaber : **HOECHST AKTIENGESELLSCHAFT**
**Postfach 80 03 20**
**D-6230 Frankfurt am Main 80 (DE)**

(72) Erfinder : **Bartels, Günter, Dr.**
**Leimenweg 31**
**D-3300 Braunschweig (DE)**

**EP 0 220 592 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung der Ketale des 4-Brombutanals und des 5-Brompentanals der Formel I

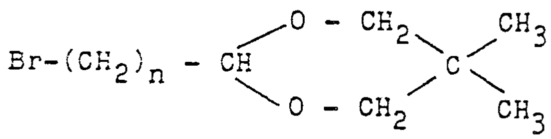 (I)

worin n = 3 oder 4 ist, sowie die letztgenannte Verbindung als solche. Bei den Verbindungen der Formel I handelt es sich um ketalisierte ω-Halogenalkanale, im folgenden auch kurz « Halogenketale » genannt, deren Grundstruktur durch Formel II beschrieben werden kann :

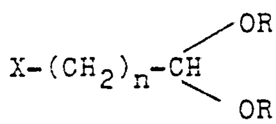 (II)

mit X = Cl, Br, J

R = Alkyl

Halogenketale der allgemeinen Struktur II mit n = 3 oder 4 sind literaturbekannt. Es sind wertvolle bifunktionelle Zwischenprodukte, die einerseits nach üblichen Reaktionen eines Alkyhalogenids, andererseits nach Spaltung der Ketalfunktion nach den vielfältigen Reaktionsmöglichkeiten eines Aldehyds umgesetzt werden können.

Anwendungsgebiete solcher Verbindungen liegen z. B. in der Synthese von Alkaloiden (C.P. Fobes et al., J. Chem. Soc. Perkin I (1977) S. 2353 ; M.E. Kuehne et al., J. Org. Chem. 1981, Bd. 46, S. 2002) und Indolderivaten (US-PS 4 252 803).

Bromketale der Formel II mit X = Br und n = 3 oder 4 wurden bisher durch Umsetzung von 4-Brombutanal bzw. 5-Brompentanal der Formel III

$$Br — (CH_2)_n — CHO \text{ mit } n = 3, 4 \qquad (III)$$

mit entsprechenden Alkoholen oder Diolen hergestellt.

Das für diese Umsetzung benötigte 4-Brombutanal bzw. 5-Brompentanal ist jedoch im technischen Maßstab nicht verfügbar. Folgende Herstellverfahren für diese beiden Verbindungen sind bekannt :

1. Partielle Reduktion von 4-Brombutyronitril bzw. 5-Brom-valeronitril zu 4-Brombutanal bzw. 5-Brompentanal mit Diisobutylaluminiumhydrid (R.D. Little, J. Org. Chem. 1982, 47, S. 362-364).

2. Oxidation der entsprechenden Halogenalkohole mit Pyridiniumchlorochromat (S.U. Kulkarni, Heterocycles 1982, 18, S. 163-167).

Die hierbei verwendeten Reagenzien sind jedoch für technische Verfahren nicht geeignet. Daher können die Bromketale der Formel I bisher im technischen Maßstab nich hergestellt werden.

Überraschenderweise wurde nun gefunden, daß diese Bromketale durch eine einfache Umwandlung aus den cyclischen Verbindungen 2,3-Dihydrofuran (IV) bzw. 3,4-Dihydro-2H-pyran (V)

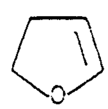 (IV) 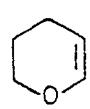 (V)

hergestellt werden können. Dazu werden diese beiden Verbindungen zunächst mit Neopentylglykol umgesetzt :

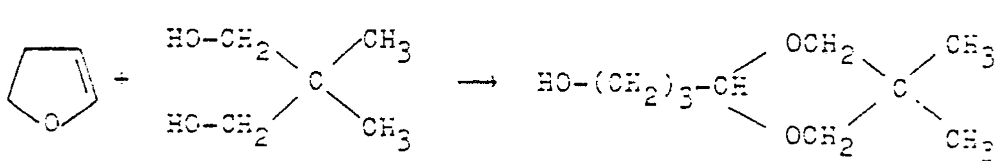

(IV) Neopentylglykol Ketal des 4-Hydroxybutanals (« Hydroxyketal »)

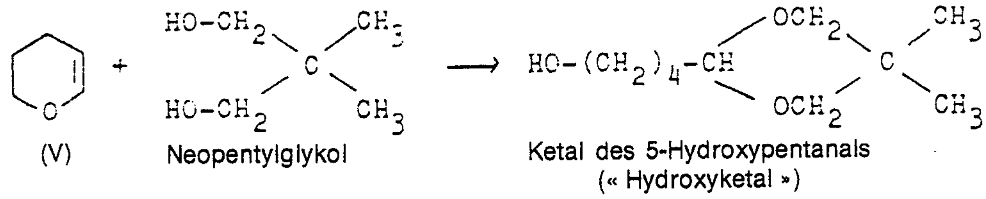

(V) Neopentylglykol Ketal des 5-Hydroxypentanals (« Hydroxyketal »)

Die entstehenden Hydroxyketale werden dann in die Bromketale übergeführt.

Die erwähnte Umsetzung des Dihydropyrans V zum entsprechenden Hydroxyketal ist bereits aus der Literatur bekannt (Synth. Communications 5 (1975), Seite 177-180). Um das entsprechende Bromketal herzustellen, wird dort jedoch zunächst das Hydroxyketal mit p-Toluolsulfonsäurechlorid und Triethylamin in das entsprechende Tosylat übergeführt, welches innerhalb von 10 Tagen mit einer Ausbeute von nur 50 % entsteht. Das Tosylat wird dann mit Lithiumbromid in das Bromketal I (mit n = 4) übergeführt, wobei die Ausbeute nur 44 % beträgt. Bezogen auf eingesetztes Dihydropyran V beträgt die Ausbeute an Bromketal I (mit n = 4) daher lediglich 22 %. Beim vorliegenden Verfahren beträgt die Ausbeute für diese Verbindung über 60 %.

Das erfindungsgemäße Verfahren zur Herstellung der Ketale des 4-Brombutanals und des 5-Brompentanals der Formel

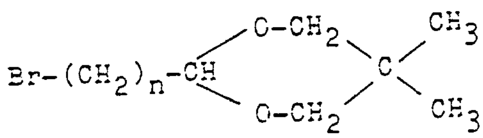

mit n = 3 oder 4

ist dadurch gekennzeichnet, daß man 2,3-Dihydrofuran bzw. 3,4-Dihydro-2H-pyran in einem Lösungsmittel in Gegenwart eines Katalysators mit Neopentylglykol umsetzt und dann das entstehende Produkt bromiert.

Vorzugsweise setzt man etwa äquimolare Mengen Neopentylglykol ein. Als Katalysatoren eignen sich alle Verbindungen, die üblicherweise bei der Überführung von Aldehyden in Ketale angewendet werden (siehe Houben-Weyl), Methoden der Organischen Chemie, Band VI/3 (1965), Seite 204 ff, insbesondere Seite 215), z. B. Salzsäure, Schwefelsäure, Sulfonsäuren, Natriumhydrogensulfat, Phosphorsäure, Phosphorpentoxid, Eisen(III)-chlorid, Zinkchlorid, Jod, wasserfreies Kupfer(II)-sulfat. Bevorzugte Katalysatoren sind Sulfonsäuren, insbesondere p-Toluolsulfonsäure.

Als Lösungsmittel eignen sich sich alle nichtprotischen Lösungsmittel, die eine ausreichende Löslichkeit der Reaktionspartner gewährleisten. Bevorzugte Lösungsmittel sind chlorierte Kohlenwasserstoffe und aromatische Kohlenwasserstoffe oder entsprechende Lösungsmittelgemische, insbesondere 1,2-Dichlorethan, Methylenchlorid und Toluol. In einer besonders bevorzugten Ausführungsform wird das Neopentylglycol zusammen mit katalytischen Mengen p-Toluolsulfonsäure in 1,2-Dichlorethan vorgelegt und Dihydrofuran (IV) bzw. Dihydropyran (V) unter Kühlung bei 0 bis 20 °C zugetropft. Anschließend läßt man bei einer Temperatur zwischen 20 °C und dem Siedepunkt des Lösungsmittels ausreagieren.

Die als Zwischenprodukte entstehenden Hydroxyketale können aus der Reaktionslösung nach Neutralisierung durch Destillation isoliert werden. Vorzugsweise setzt man sie jedoch in der Reaktionslösung, d. h. ohne sie zu isolieren, mit einem Bromierungsreagenz weiter um.

Als Bromierungsreagenz eignen sich alle Verbindungen, die üblicherweise zur Überführung von Alkoholen in Alkylbromide eingesetzt werden und den Erhalt der Ketalfunktion gewährleisten (siehe Houben-Weyl, Band V/4 (1960), Seite 361 ff). Hierzu zählen z. B. Phosphorbromide und $SOBr_2$.

Bei Verwendung von Phosphortribromid wird nach bekannter Verfahrensweise (siehe Houben-Weyl, Band V/4 (1960), Seite 389, 390) zunächst unter Kühlung bei 0-10 °C das Phosphorbromid eingetropft und anschließend zum Siedepunkt des Lösungsmittels erhitzt, bis zur vollständigen Umsetzung. Das nach üblicher Aufarbeitung (Houben-Weyl, Band V/4 (1960), Seite 389, 390) erhaltene Rohprodukt wird durch Destillation im Vakuum gereinigt. Die Bromketale werden in einer Ausbeute von 60-70 % erhalten.

## Beispiel 1

2-(4-Brombutyl)-5,5-dimethyl-1,3-dioxan (= I mit n = 4)

In eine Suspension von 833 g Neopentylglycol in 2,5 l 1,2-Dichlorethan wurden nach Zusatz von 8 g p-Toluolsulfonsäure bei Raumtemperatur innerhalb von 60 Minuten 673 g 3,4-Dihydro-2H-pyran eingetropft. Nach einer Reaktionszeit von 16 Stunden bei Raumtemperatur kühlte man auf 0 °C ab und tropfte innerhalb von 1,5 Stunden 758 g Phosphortribromid ein. Man ließ 3 Stunden bei 0 °C und 3 Stunden bei 80 °C reagieren. Die Reaktionslösung wurde nach Abkühlen auf Raumtemperatur mit Wasser und Carbonat-Lösung gewaschen und eingedampft. Aus dem Rohprodukt erhielt man durch Destillation im Hochvakuum 1 250 g Produkt (62 % d. Th.) in einer Reinheit von 94 % (GC).

Beispiel 2

2-(3-Brompropyl)-5,5-dimethyl-1,3-dioxan (= I mit n = 3)

In eine Suspension von 833 g Neopentylglycol in 2,5 l 1,2-Dichlorethan wurden nach Zusatz von 8 g p-Toluolsulfonsäure bei Raumtemperatur innerhalb von 60 Minuten 561 g 2,3-Dihydrofuran eingetropft. Nach einer Reaktionszeit von 16 Stunden bei Raumtemperatur kühlte man auf 0 °C ab und tropfte innerhalb von 1,5 Stunden 758 g Phosphortribromid ein. Man ließ 3 Stunden bei 0 °C und 3 Stunden bei 80 °C reagieren. Die Reaktionslösung wurde nach Abkühlen auf Raumtemperatur mit Wasser und Carbonat-Lösung gewaschen und eingedampft. Aus dem Rohprodukt erhielt man durch Destillation im Hochvakuum 1 270 g Produkt (67 % d. Th.) in einer Reinheit von 93 % (GC).

Siedepunkt 79 °C bei 0,13 mbar

NMR-Spektrum δ ($CDCl_3$) :

0,72 ppm (s) 3H

1,17 ppm (s) 3H

1,4-2,3 ppm (m) 4H

3,2-3,8 ppm (m) 6H

4,44 ppm (t, 5Hz) 1H

**Patentansprüche**

1. Verfahren zur Herstellung der Ketale des 4-Brombutanals und des 5-Brompentanals der Formel

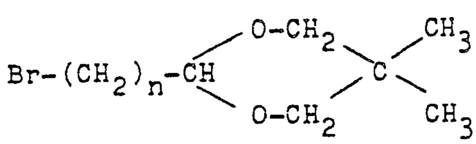

(I)

mit n = 3 oder 4

dadurch gekennzeichnet, daß man 2,3-Dihydrofuran bzw. 3,4-Dihydro-2H-pyran in einem Lösungsmittel in Gegenwart eines Katalysators mit Neopentylglykol umsetzt und dann das entstehende Produkt bromiert.

2. Verfahren nach Anspruch 1, dadurch gekennzeichnet, daß man etwa äquimolare Mengen Neopentylglykol einsetzt.

3. Verfahren nach Anspruch 1 oder 2, dadurch gekennzeichnet, daß man als Katalysator eine Sulfonsäure einsetzt.

4. Verfahren nach einem der Ansprüche 1 bis 3, dadurch gekennzeichnet, daß man die Bromierung mit einem Phosphorbromid oder $SOBr_2$ vornimmt.

5. 2-(3-Brompropyl)-5,5-dimethyl-1,3-dioxan.

**Claims**

1. A process for preparing the ketals of 4-bromo-butanal and of 5-bromopentanal of the formula

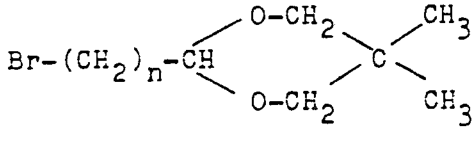

(I)

with n = 3 or 4

which comprises reacting 2,3-dihydrofuran and 3,4-dihydro-2H-pyran respectively in a solvent in the presence of a catalyst with neopentylglycol and then brominating the resulting product.

2. The process as claimed in claim 1, wherein approximately equimolar amounts of neopentylglycol are used.

3. The process as claimed in claim 1 or 2, wherein the catalyst used is a sulfonic acid.

4. The process as claimed in any one of claims 1 to 3, wherein the bromination is effected with a phosphorus bromide or $SOBr_2$.

5. 2-(3-Bromopropyl)-5,5-dimethyl-1,3-dioxane.

**Revendications**

1. Procédé pour préparer les acétals du bromo-4 butanal et du bromo-5 pentanal qui répondent à la formule

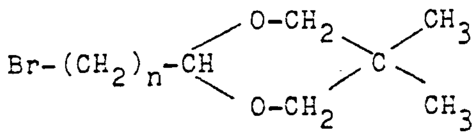

(I)

dans laquelle n est égal à 3 ou 4, procédé caractérisé en ce qu'on fait réagir le dihydro-2,3 furanne ou le dihydro-3,4 2H-pyranne, dans un solvant, en présence d'un catalyseur, avec le néopentylglycol, puis on brome le produit formé.

2. Procédé selon la revendication 1 caractérisé en ce que le néopentylglycol est mis en jeu en une quantité à peu près équimolaire.

3. Procédé selon l'une des revendications 1 et 2, caractérisé en ce qu'on utilise, comme catalyseur, un acide sulfonique.

4. Procédé selon l'une quelconque des revendications 1 à 3, caractérisé en ce qu'on effectue la bromation avec un bromure de phosphore ou avec $SOBr_2$.

5. (Bromo-3 propyl)-2 diméthyl-5,5 dioxanne-1,3.